(19) Europäisches Patentamt
European Patent Office
Office européen des brevets

(11) Numéro de publication: **0 148 681**
**B1**

(12) # FASCICULE DE BREVET EUROPEEN

(45) Date de publication du fascicule du brevet: **24.06.87**

(21) Numéro de dépôt: **84402595.7**

(22) Date de dépôt: **14.12.84**

(51) Int. Cl.[4]: **C 09 B 63/00**, C 09 B 61/00, D 06 P 1/673, D 06 P 1/34, D 06 P 3/30, D 21 H 3/80, A 61 K 7/13, C 09 D 5/00, C 09 D 11/00

(54) **Procédé de teinture avec des colorants en poudre et composition pour sa réalisation.**

(30) Priorité: **21.12.83 FR 8320447**
**04.12.84 FR 8418430**

(43) Date de publication de la demande:
**17.07.85 Bulletin 85/29**

(45) Mention de la délivrance du brevet:
**24.06.87 Bulletin 87/26**

(84) Etats contractants désignés:
**AT CH DE GB IT LI NL SE**

(56) Documents cités:
**DE - C - 472 975**
**FR - A - 618 981**
**FR - A - 812 944**
**FR - A - 1 472 456**
**FR - A - 2 030 364**
**FR - A - 2 154 769**
**FR - A - 2 432 035**
**GB - A - 247 328**
**US - A - 3 340 000**

(73) Titulaire: **SECTA-LABORATOIRES DE COSMETOLOGIE YVES ROCHER, 6 Avenue Kléber, F-75116 Paris (FR)**

(72) Inventeur: **Melin-Moulet, Anne-Marie, 361 rue aux Chiens, F-45160 Olivet (FR)**

(74) Mandataire: **Kohn, Armand, 5 Avenue Foch, F-92380 Garches (FR)**

**EP 0 148 681 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

L'invention concerne un procédé de teinture et une composition colorante pour sa réalisation. L'invention est applicable à la teinture de diverses matières, notamment fibres textiles, feuilles cellulosiques, polymères, matières kératiniques, et autres.

Bien que la teinture soit une technique ancienne, elle comporte des difficultés et aléas qui se présentent souvent avec différentes matières colorantes, surtout quand celles-ci sont employées sous la forme pulvérulente. La présente invention remédie aux irrégularités de teintures quel que soit le colorant employé, et elle est particulièrement utile pour les colorants naturels.

L'utilisation de colorants végétaux est connue et pratiquée depuis l'antiquité. La teinture était faite par la mise en contact de la matière à teindre avec une suspension aqueuse de différentes parties du végétal riche en colorant; mais les résultats n'étaient pas reproductibles, à cause des variations des teneurs et qualités du colorant d'un lot de plantes à l'autre; d'autre part, l'hétérogénéité du bain conduit à des irrégularités de colorant sur la matière traitée.

Les extraits végétaux concentrés sous la forme liquide, à l'état de blocs, en poudre cristallisée ou en poudre atomisée, pour teinture des textiles, des cuirs ou encres, ne donnent une coloration reproductible que dans des opérations portant sur des grandes quantités de colorants et de matières à teindre.

Cependant, étant donné la non toxicité de la plupart des colorants naturels, ainsi que le prix très abordable de certains d'entre eux, ces colorants ont encore, à l'heure actuelle, de nombreuses applications. Ils sont utilisés dans les industries alimentaire et pharmaceutique, par exemple pour la coloration de boissons, huiles, produits laitiers, confiserie, biscuits ou glaces. Mais leurs extraits, même très concentrés, subissent, le plus souvent, des dégradations plus ou moins poussées après quelques mois de stockage. A noter également que les couleurs pratiquement possibles sont limitées au jaune, à l'orange, au rouge, au vert et au brun.

En ce qui concerne l'application particulière des extraits colorants végétaux à la coloration capillaire, elle est simple, mais la coloration obtenue est faible et la conservation des extraits aléatoire.

Certains compositions, pour la teinture des cheveux, comprennent un extrait colorant, végétal, sec, une poudre de plante colorante, ou bien le colorant lui-même en poudre, en mélange avec des substances telles que sucres, polyols, sels minéraux ou organiques ou poudres de végétaux épuisés. Au moment de l'emploi, l'ensemble est mélangé avec du lait tiède ou avec de l'eau tiède et placé sur la chevelure en vue de l'obtiention de reflets. Ces genres de préparations sont difficiles d'emploi, parce qu'ils forment une pâte très épaisse ou emplâtre; ils exigent de fortes quantités de colorant et des moyens mécaniques pour la préparation destinée à la chevelure.

Il existe également des mélanges de poudres de végétaux colorants, tout prêts pour être mis sous la forme de cataplasmes par addition d'eau, en vue de la coloration d'une chevelure. Ces produits présentent des difficultés d'emploi inhérentes à la formation d'un cataplasme; en général, la coloration atteinte est faible et le temps d'application long.

Certains colorants, utilisés dans des peintures, enduits, vernis, encres ou matières plastiques, se trouvent sous la forme de pigments ou de laques; le colorant y est adsorbé sur une poudre insoluble ou combiné à celle-ci à l'état d'un complexe métallique. Il est donc rendu insoluble et n'est pas libéré lorsque le pigment ou la laque est délayé dans l'eau. Dans les utilisations susindiquées, ce produit se trouve en dispersion dans un milieu renfermant un liant tel que résine, gomme ou polymère synthétique; lorsqu'on applique cette dispersion sur un support, le colorant ne réagit pas avec celui-ci; autrement dit, il n'y est pas question de teinture du support par le colorant.

Ainsi, alors que la teinture en général peut présenter des difficultés, celle qui utilise des colorants naturels, végétaux, est particulièrement délicate. Cette technique présente des défauts d'incommodité des opérations, du manque de reproductivité, d'une intensité de couleur généralement trop faible, et des difficultés de conservation de la préparation colorante.

On connaît un ancien procédé de teinture, selon FR 618 981, exécuté en présence de terre à foulons ou de produits semblables, susceptibles d'éliminer la graisse ou du colorant mal fixé. D'après FR 812 944 on prépare des épaississants pour impressions ou apprêts, en ajoutant aux gommes connues des produits du genre de la bentonite, de formule générale 4 $SiO_2 \cdot Al_2O_3 \cdot nH_2O$.

La présente invention apporte à la teinture un progrès important; elle permet l'obtention de préparations de colorants, très stables, aisément applicables à la teinture de toutes sortes de matières, à petite ou à grande échelle. L'invention rend possible également la préparation de compositions de toute concentration voulue, applicables à l'obtention des teintes légères, aussi bien que des colorations intenses. Les compositions, suivant l'invention, sont caractérisées par une très bonne stabilité, dans les conditions courantes de conservation, notamment à la température ambiante, à l'obscurité, dans un flacon bouché sans précaution particulière. Ces produits sont insensibles à l'humidité et n'exigent l'adjonction d'aucun conservateur; un extrait végétal assure plus efficacement sa protection qu'un conservateur, dans certains cas.

La présente invention concerne un procédé de teinture, dans lequel le support à teindre est mis en contact avec un bain auquel on a ajouté un colorant pulvérulent et une poudre de matière inorganique, insoluble dans le bain, caractérisé en ce que l'on choisit un colorant et une matière inorganique de telle sorte que celle-ci soit chimiquement inerte vis-à-vis du colorant, ne l'absorbe pas ne donne pas de combinaison colorée insoluble avec lui, que la poudre de colorant et celle de la matière inorganique sont intimement mélangées, en particules d'environ 1 à 50 µm, et que la poudre de la matière inorganique présente une surface spécifique comprise entre 4 et 110 $m^2/g$.

La matière inorganique peur être introduite dans le bain de teinture séparément de l'introduction du colorant, après quoi le bain est soigneusement mélangé; il est cependant préférable d'utiliser un mélange très intime de ces deux substances, préparé d'avance.

L'inertie du composé inorganique vis-à-vis du colorant signifie que, placés en milieu solvant ces produits ne forment aucune combinaison insoluble du colorant. Autrement dit, lorsque la composition suivant l'invention est délayée avec du solvant on retrouve dans le liquide les particules inchangées de la matière inorganique et les particules ou solution du colourant. C'est là une différence fondamentale entre les compositions suivant l'invention et les pigments bien connus dans la technique, qui comportent des particules formées par un composé minéral auquel est combiné un colorant, ces particules étant insolubles dans le solvant. En outre, le composé inorganique ne doit donner lieu à aucune réaction d'oxydation, de réduction ou de libération d'ions vis-à-vis du colorant utilisé.

Dans le cas d'un colorant naturel, la poudre, utilisée suivant l'invention, est de préférence constituée par un extrait fait à la manière connue en soi, à partir d'une ou de plusieurs parties d'une plante renfermant le colorant; la forme préférée de l'invention utilise une poudre de la matière colorante, végétale, elle-même. Cependant, le fait d'ajouter le composé inorganique permet l'obtention de bons produits colorants, même avec des poudres obtenues par le broyage de racines, feuilles, pétales, fruits, écorces, bois, graines, etc. d'une plante colorifère; grâce à la finesse du broyage et à la présence du composé inorganique les inconvénients, signalés plus haut au sujet des anciennes méthodes, ne se font plus sentir ici.

Dans la réalisation de l'invention, la finesse des poudres employées doit être telle que les particules présentent de préférence une taille de 2 à 30 µm. Des résultats particulièrement favorables sont obtenus lorsque les particules du colorant ont des tailles comprises entre 5 et 10 µm et celles de la matière inorganique de 2 à 16 µm.

Le premier critère, dans le choix du composé inorganique, est — comme indiqué plus haut — de ne pas former de combinaison insoluble dans le solvant avec le colorant voulu. Les deux autres critères résident, de préférence, en une densité apparente ne dépassant pas 300 $g/dm^3$ ou mieux comprise entre 50 et 200 $g/dm^3$ pour la poudre non tassée. La surface spécifique est de préférence inférieure à 8 $m^2/g$.

A titre d'exemple, on peut citer, en tant que composé inorganique convenant à la réalisation de l'invention, des oxydes de zinc, titane, silicium ou Al, des silicates naturels comme kaolin, bentonite ou talc, certains carbonates, phosphates ou fluorures comme ceux de calcium ou de magnésium, des silicoaluminates complexes comme le mica, par exemple. En dehors des silices naturelles, on peut employer les silices synthétiques, microcristallines, hydrophiles ou rendues hydrophobes par un traitement chimique.

Il est bien entendu qu'un composé inorganique qui, avec certains colorants, donne un pigment et par conséquent ne fait pas partie de l'invention pour ce colorant là, peut éventuellement être utilisé, dans le cadre de la présente invention — avec un autre colorant — avec lequel il ne donne pas de pigment insoluble. C'est par exemple le cas de l'alumine hydratée qui forme, avec l'extrait de garance ou avec l'alizarine, une laque qui est en dehors de la présente invention; par contre, la même alumine hydratée peut former un mélange, suivant l'invention, avec un extrait de feuilles d'indigo ou avec le violet de méthyle.

Les proportions relatives de colorant et de composé inorganique peuvent varier dans une large limite, notamment de 5 à 98% de l'un d'entre eux, dans le mélange; pratiquement, les porportions préférées sont d'environ 10 à 80%, en poids.

Il va de soi qu'une composition suivant l'invention peut comprendre un ou plusieurs colorants avec un ou plusieurs composés inorganiques définis plus haut.

Les colorants, mis sous la forme des compositions suivant l'invention, sont particulièrement stables au stockage pendant un temps assez long, dépassant, dans la plupart des cas, 18 mois, même lorsque la teneur en élément colorant est faible. De plus, ils sont facilement dispersés ou remis en solution, de manière uniforme, sans formation d'amas, de grumeaux ou de micelles, dans le solvant servant habituellement à réaliser la teinture proprement dite, selon la matière à teindre; cela peut se faire par simple agitation, ne nécessitant l'emploi d'aucun appareillage spécial, et cela aussi bien à la température ambiante qu'à toute température comprise entre 10°C et la température d'ébullition du solvant, suivant le processus exigé par la nature de la matière à colorer. Le solvant peut être de l'eau, un autre solvant, un mélange de solvants ou bien une solution constituée par différentes substances chimiques généralement utilisées pour les teintures.

Grâce à la préparation colorante, réalisée suivant l'invention, la teinture de fibres ou d'autres matières peut se faire de manière tout à fait homogène; elle est très simple et la reproductivité des teintes est facilitée, parce que le procédé de l'invention rend aisée la manipulation de très petites quantités de substances colorantes, de façon précise, sans matériel de pesage compliqué ou élaboré. En effet, il suffit de prendre une composition suivant l'invention à faible teneur en colorant, pour que la quantité de matière colorante soit faible pour un volume relativement grand. De plus, grâce aux composés suivant l'invention, les molécules de colorant peuvent réagir de manière uniforme avec les sels métalliques, les substances oxydantes ou autres réactifs présents dans la solution de teinture: on obtient ainsi une matière teinte de façon homogène en tous points. Par conséquent, l'utilisateur peut disposer facilement de plusieurs nuances pour une même teinte, et cela sans faire varier son procédé de teinture pour une même matière à teindre: l'intensité de la coloration est déterminée par la fabrication même de la composition colorante. En outre, les différentes teintes, pouvant être proposées, sont mélangeables sans inconvénient, lorsqu'il n'y a pas de réactivité entre les colorants utilisés, avant la teinture proprement dite; les différents colorants se trouvent alors répartis uniformément sur la matière à teindre et l'utilisateur peut obtenir des teintes très variées.

Un autre avantage du procédé suivant l'invention réside en ce que le temps, nécessaire à l'obtention de la teinture d'une matière donnée, est extrêmement réduite. Par exemple, dans le cas de la laine, les teintures à l'aide de végétaux, pour lesquelles il fallait deux jours, voire davantage, peuvent être réalisées en seulement 2 à 3 heures.

Le procédé de l'invention convient à la teinture de divers supports à l'aide de colorants inorganiques ou organiques, notamment des matières d'origine animale, notamment pourpre de Tyr, carmin de Cochenille ou la guanine, et des nombreuses matières colorants synthétiques, comme, par exemple, les azoïques, phtalocyanines, oxazines, thiazines, anthraquinoniques, benzidiniques, xanthéniques et autres. Ainsi peut-on appliquer l'invention à la teinture avec divers colorants pour vernis, matières plastiques, textiles, papier, bois, cuir, pour encres d'imprimerie, produits pharmaceutiques, alimentaires ou cosmétiques.

L'invention est particulièrement utile à la teinture au moyen de colorants naturels; elle s'applique à un grand nombre de plantes colorantes, et permet l'obtention d'une large gamme de teintes de base, notamment jaune, brun, orange, bleu, rouge, noir et, par mélange, des teintes intermédiaires. A titre d'exemple, on donne ci-après un tableau de quelques-unes des plantes pouvant être utilisées pour l'obtention des extraits colorants employés dans le procédé suivant l'invention.

| *Nom de la plante* | *Parties utilisées* |
|---|---|
| Camomille des teinturiers, allemande, romaine | Capitules floraux |
| Gaude-Réséda | Plante |
| Safran | Stigmates de fleurs |
| Bois d'Arc | Bois |
| Quercitron | Ecorce et bois |
| Sophora japonica | Boutons floraux |
| Mûriers des teinturiers | Bois |
| Nerprun des teinturiers | Fruits |
| Bourdaine | Ecorce |
| Rhubarbe de Chine | Rhizome |
| Souci | Fleurs |
| Kesu | Fleurs |
| Curcuma | Rhizome |
| Fustet | Ecorce et bois |
| Acacia catechu, cachou, gambir, noix d'Arec | |
| Noyer | Fruit |
| Rocou | Graine |
| Henné | Feuille |
| Indigo | Feuille |
| Garance | Racine |
| Morinda | Racine |
| Orcanette | Racine |
| Santal rouge | Bois |
| Pernambouc | Bois |
| Lichens | Plante |
| Sang-Dragon | Sécrétion des fruits |
| Campêche | Bois |

Les exemples qui suivent illustrent l'invention.

*Exemple 1*

*Coloration du papier*

Dans 100 parties d'une suspension de 2,5 parties de pâte de bois bisulfitée, blanchie dans l'eau, on ajoute: 0,08 parties de sulfate d'aluminium cristallisé, préalablement dissous dans 0,8 parties d'eau. A cette préparation, est additionné, sous agitation, 0,25 g d'une composition comprenant, selon l'invention:

10% d'un extrait de campêche
85% de talc en particules de 2 à 5 µm
5% de silice microcristalline, de 2 à 4 µm.

Au bout de 15 minutes, l'ensemble est filtré sur toile, pressé puis séché. La feuille de papier, ainsi réalisée, est colorée dans la masse en un beau violet uniforme; de plus, la coloration résiste bien à l'eau.

Si des extraits colorants simples sont utilisés, il en résulte une feuille de papier présentant un fond bleu-violet moucheté de façon irrégulière de tâches violet plus foncé.

*Exemple 2*

*Coloration du papier*

Les opérations sont les mêmes qu'à l'exemple 1, mais la composition colorante esr remplacée par la suivante:

4% d'extrait de campêche,
6% d'extrait de Sophora japonica,
85% de talc,
5% de silice microcristalline.

La feuille de papier obtenue est unifomément verte.

*Exemple 3*

*Teinture des fourrures*

Sur une toison de mouton, lavée et tannée, est appliquée à l'aide d'une brosse douce, sur le sommet des poils, une solution d'acétate ferreux. Après séchage, la solution suivante est appliqué:

100 parties d'eau de chaux (eau saturée en hydroxyde de calcium)
2 parties de sulfate double d'aluminium et d'ammonium
2 parties de sulfate de fer
10 parties, ajoutées juste au moment de l'emploi, d'une composition constituée selon l'invention par:
27% de kaolin en particules de 3 à 10 µm
3% de silice microcristalline
25% d'extrait de campêche
45% d'extrait de pernambouc.

L'ensemble de cette solution, appliquée également à la brosse, sert à colorer uniformément la toison en brun, si cette dernière est entièrement recouverte. Si elle est répartie par taches, on obtient une imitation de la fourrure de certains animaux.

Ce procédé a été également expérimenté sur d'autres types de toisons ou fourrures.

Lorsqu'on veut effectuer une teinture semblable de fourrures par la méthode ancienne, utilisant une poudre de plantes, on se heurte à des difficultés. Il en est de même quand on veut employer un extrait végétal non préparé suivant l'invention; en effet, la solution de teinture n'est pas homogène, elle forme une suspension avec des amas de particules de colorant, dus à la mauvaise solubilisation, surtout à 20°C. La coloration des fourrures est irrégulière, inacceptable, à moins d'avoir recours à une très longue et puissante agitation.

*Exemple 4*

*Teinture des fils de laine*

100 parties de laine en écheveaux sont traitées dans une solution contenant:

10 parties de sulfate double d'aluminium et de potassium
3 parties d'hydrogénotartrate de potassium
3000 parties d'eau

pendant une heure à 90°C.

Après refroidissement, rinçage et égouttage, la teinture est réalisée dans

3000 parties d'eau dans laquelle sont ajoutées 10 parties de la composition suivante, préparée selon l'invention:
70 parties d'un extrait de genêt des teinturiers
27 parties de talc
3 parties de silice microcristalline.

Après 45 minutes à 90°C, les fils sont colorés en un beau jaune citron, uniforme et résistant.

Dans le cas où les plantes ou la poudre de plante sont utilisées, 2 jours sont nécessaires pour réaliser la teinture et celle-ci exige le mode opératoire laborieux, suivant: 500 parties de rameaux jeunes de genêt des teinturiers, broyés, sont mis à macérér une nuit dans 3000 parties d'eau non calcaire, puis portées à ebullition pendant 45 minutes. Après refroidissement, la solution est filtrée sur toile fine et les résidus végétaux enfermés dans un sac de toile, puis remis dans le bain de teinture. Les écheveaux de laine, mordancés comme précédemment et encore humides, sont alors placés dans cette solution et l'ensemble porté à 90°C pendant 45 minutes. Après séchage, les fils sont colorés en jaune citron. Toutefois, si la solution de teinture n'est pas convenablement filtrée, le colorant n'est pas uniforme et de nombreux rinçages, après refroidissement, sont nécessaires, afin d'éliminer totalement la poudre végétale.

*Exemple 5*

*Teinture capillaire*

Suivant la technique décrite dans la demande de brevet français n° 83 12458, les cheveux sont tout d'abord traités par une préparation (A) contenant

5% de nonylphénol à 10 moles d'oxyde d'éthylène,
0,8% de diéthanolamide de l'huile de coprah
3% de lactate d'aluminium
q.s. % d'eau désionisée portée à pH 3,5 avec de l'acide lactique.

Après 5 minutes de ce traitement, les cheveux sont rincés puis on leur applique la préparation suivante (B):

3,5 parties de nonylphénol à 6 moles d'oxyde d'éthylène
1 partie de monooléate de sorbitan
1,5 parties d'isopropanol
2 parties d'acide stéarique
0,3 partie de soude
1 partie d'alginate de sodium

à laquelle sont ajoutées 3 parties d'une composition contenant selon l'invention:

30% de kaolin
3% de silice microcristalline
30% d'extrait de santal rouge
20% d'extrait de rocou
17% d'extrait de campêche.

Appliquée pendant 30 minutes sur des cheveux blonds, cette composition permet d'obtenir des cheveux châtains à reflets auburn, intense, après un simple rinçage et séchage.

L'utilisation uniquement d'extraits végétaux conduit à une solution (B) non homogène; il y a agglomération des colorants par paquets dans la préparation, et, par suite, la coloration est non uniforme.

L'emploi direct de poudre de ces végétaux, quant à lui, conduit à une solution (B) d'application difficile, formant emplâtre et ne permet l'obtention que de très pâles reflets, non homogènes du point de vue de la coloration et de la répartition. De plus, d'abondants rinçages de la chevelure sont nécessaires à l'élimination des poudres végétales.

*Exemple 6*

*Vernis teintant pour le bois*

On prépare un mélange contenant:

14 parties de gomme laque
7 parties de colophane
3 parties de térébenthine
76 parties d'alcool éthylique.

On ajoute 1,6 parties d'une composition suivant l'invention contenant:

50 parties de colorant CI acide brun 357
25 parties de talc à particules de 2 à 5 µm
25 parties de kaolin micronisé.

Par application au pinceau sur un bois blanc, on obtient une belle teinte brune, vernissée sans marbrures ni picots.

L'utilisation directe du même colorant conduit à une teinte marbrée et tâchetée.

*Exemple 7*

*Encre colorée*

Dans 85 parties d'un mélange en poids égaux distillée et d'alcool éthylique, on dissout 15 parties d'une résine de polyamide 6 à bas poids moléculaire. On ajoute ensuite 0,5 parties d'une composition suivant l'invention comprenant:

60 parties d'un colorant jaune à complexe métallifère de chrome (C.I. acide jaune 121)
10 parties de silice microcristalline de 2 à 4 µm
30 parties de kaolin de 5 à 8 µm.

Par application au pochoir sur un flacon de polyéthylène suivie d'un séchage, on obtient des motifs décoratifs jaunes très brillants, dépourvus de toutes marbrures.

*Exemple 8*

Dans une machine de teinture textile permettant de teindre le polyester par le procédé dit à circulation de bain à haute température, on introduit une bobine de jersey de polyester texturé avec un rapport de bain de 1 pour 10.

Pour 100 parties de polyester, on empâte, dans une petite fraction du bain de teinture 1,5 parties d'une préparation selon l'invention comprenant:

30% d'un colorant rouge de formule

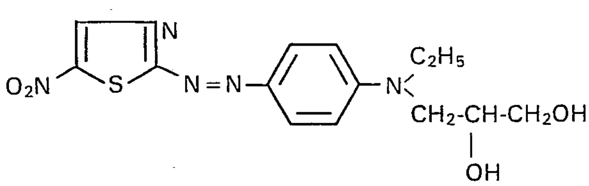

50% de silice microcristalline
20% de talc.

Le produit empâté est introduit dans le bain de teinture en circulation à 30°C. Puis l'appareil est fermé et la température portée à 120°C pendant une heure. Après refroidissement et lavage, on obtient une pièce de tissu uniformément teinte en un beau coloris rouge soutenu.

L'utilisation du colorant rouge en poudre tel quel est difficile car il s'empâte mal dans le bain de teinture et donne des taches sur le textile.

*Exemple 9*

100 parties de tissus de soie sont traités pendant une heure à 50°C dans un bain composé de:
3000 parties d'eau
10 parties de sulfate double d'aluminium et de potassium
3 parties d'hydrogénotartrate de potassium.

Après refroidissement, rinçage et égouttage, la teinture est réalisée dans 2000 parties d'eau dans lesquelles sont ajoutées 10 parties de la composition suivante:

50 parties d'un extrait de cochenille
40 parties de kaolin
10 parties de silice microcristalline.

La température est portée à 50°C pendant une heure, puis le bain de teinture est refroidi lentement. Quand il est revenu à température ambiante, on sort le tissu de soie, le rince, et le met à sécher. Il est d'une couleur carmin intense, lumineuse et parfaitement uniforme.

**Revendications**

1. Procédé de teinture, dans lequel le support à teindre est mis en contact avec un bain auquel on a ajouté un colorant pulvérulent et une poudre de matière inorganique, insoluble dans le bain, caractérisé en ce que l'on choisit un colorant et une matière inorganique de telle sorte que celle-ci soit chimiquement inerte vis-à-vis du colorant, ne l'absorbe pas, ne donne pas de combinaison colorée insoluble avec lui, que la poudre de colorant et celle de la matière inorganique sont en particules d'environ 1 à 50 µm, qu'elles sont intimement mélangées et que la poudre de la matière inorganique présente une surface spécifique comprise entre 4 et 110 $m^2/g$.

2. Procédé suivant la revendication 1, caractérisé en ce que la poudre de colorant et celle de la matière inorganique sont en particules de 2 à 30 µm.

3. Procédé suivant la revendication 1 ou 2, caractérisé en ce que la matière inorganique est en poudre dont la taille des particules est de 2 à 16 µm.

4. Procédé suivant une des revendications 1 à 3, caractérisé en ce que les particules du colorant ont des tailles comprises entre 5 et 10 µm.

5. Procédé suivant une des revendications 1 à 4, caractérisé en ce que la poudre de la matière inorganique a une densité apparente ne dépassant pas 300 $g/dm^3$ et de préférence comprise entre 50 et 200 $g/dm^3$ à l'état non tassé.

6. Procédé suivant une des revendications précédentes, caractérisé en ce que la poudre de la matière inorganique présente une surface spécifique inférieure à 8 $m^2/g$.

7. Procédé suivant une des revendications précédentes, caractérisé en ce que la matière inorganique est un oxyde ou silicate d'un métal, en particulier du zinc, titane ou silicium, aluminium, notamment un silicate naturel du type kaolin, bentonite, talc, et silice.

8. Procédé suivant une des revendications 1 à 6, caractérisé en ce que le composé minéral est une silice microcristalline, hydrophile ou rendue hydrophobe.

9. Procédé suivant une des revendications précédentes, caractérisé en ce que le colorant est une matière colorante organique, synthétique, ou animale.

10. Procédé suivant une des revendications précédentes, caractérisé en ce que le colorant est un extrait de plante colorifère.

11. Composition, pour la réalisation du procédé, suivant une des revendications 1 à 10, caractérisé en ce qu'elle est constituée par un mélange intime de 5 à 98% en poids de colorant, sec, et de respectivement 95 à 2% de matière inorganique, les deux composants étant en particules de 1 à 50 µm.

12. Composition suivant la revendication 11, caractérisée en ce qu'elle se compose seulement de 10 à 80% de colorant sec en poudre dont les particules sont de 2 à 30 microns, et de respectivement 90 à 20% de matière inorganique en particules de 2 à 30 µm.

**Patentansprüche**

1. Färbeverfahren, bei dem der zu färbende Träger in Kontakt mit einem Bad gebracht wird, in das ein pulverförmiges Färbemittel und ein Pulver eines in dem Bad unlöslichen anorganischen Materials gefügt wurde, dadurch gekennzeichnet, dass man ein Färbemittel und ein anorganisches Material von der Art auswählt, dass es gegen dem Färbemittel chemisch inert ist, es nicht absorbiert, mit diesem keine unlösliche gefärbte Kombination ergibt, dass das Pulver des Färbemittels und des anorganischen Materials in Teilchen von ungefähr 1 bis 50 µm vorliegen, dass sie innig vermischt sind und dass das Pulver des anorganischen Materials eine spezifische Oberfläche von 4 bis 110 $m^2/g$ hat.

2. Verfahren nach Anspruch 1, dadurch gekennzeichnet, dass das Pulver des Färbemittels und das des anorganischen Materials in Teilchen von 2 bis 30 µm vorliegen.

3. Verfahren nach Anspruch 1 oder 2, dadurch gekennzeichnet, dass das anorganische Material in Pulverform ist, dessen Teilchengrösse 2 bis 16 µm beträgt.

4. Verfahren nach einem der Ansprüche 1 bis 3, dadurch gekennzeichnet, dass die Teilchen des Färbemittels Grössen von 5 bis 10 µm haben.

5. Verfahren nach einem der Ansprüche 1 bis 4, dadurch gekennzeichnet, dass das Pulver des anorganischen Materials eine scheinbare Dichte, die 300 $g/dm^3$ nicht überschreitet, und vorzugsweise zwischen 50 und 200 $g/dm^3$, im nicht-komprimierten Zustand, hat.

6. Verfahren nach einem der vorhergehenden Ansprüche, dadurch gekennzeichnet, dass das Pulver des anorganischen Materials eine spezifische Oberfläche von unter 8 $m^2/g$ hat.

7. Verfahren nach einem der vorhergehenden Ansprüche, dadurch gekennzeichnet, dass das anorganische Material ein Oxid oder Silicat eines Metalls, insbesondere von Zink, Titan oder Silicium, Aluminium, insbesondere ein natürliches Silicat des Types Kaolin, Bentonit, Talkum und Siliciumdioxid, ist.

8. Verfahren nach einem der Ansprüche 1 bis 6, dadurch gekennzeichnet, dass die mineralische Verbindung ein mikrokristallines, hydrophiles oder hydrophob gemachtes Siliciumdioxid ist.

9. Verfahren nach einem der vorhergehenden Ansprüche, dadurch gekennzeichnet, dass das Färbemittel ein organisches, synthetisches oder tierisches färbendes Material ist.

10. Verfahren nach einem der vorhergehenden Ansprüche, dadurch gekennzeichnet, dass das Färbemittel einfarbiger Pflanzenextrakt ist.

11. Zusammensetzung zur Durchführung des Verfahrens nach einem der Ansprüche 1 bis 10, dadurch gekennzeichnet, dass sie aus einem innigen Gemisch von 5 bis 98 Gew.-% trockenem Färbemittel und jeweils 95 bis 2% anorganischem Material besteht, wobei die beiden Bestandteile in Teilchen von 1 bis 50 µm vorliegen.

12. Zusammensetzung nach Anspruch 11, dadurch gekennzeichnet, dass sie sich nur aus 10 bis 80% trockenem pulverförmigem Färbemittel, dessen Teilchen 2 bis 30 µm betragen, und jeweils 90 bis 20% anorganischem Material in Teilchen von 2 bis 30 µm zusammensetzt.

**Claims**

1. Process of dyeing, in which the substrate to be dyed is contacted with a bath to which a pulverulent dye and a powder of an inorganic material, insoluble in the bath, have been added, characterised in that a dye and an inorganic material are selected such that the latter is chemically inert to the dye, does not absorb it, does not give an insoluble coloured combination with it, the powder of the dye and that of the inorganic material are particles of about 1 to 50 µm, they are intimately mixed and the powder of the inorganic material has a specific surface in the range from 4 to 110 $m^2/g$.

2. Process according to claim 1, characterised in that the powder of the dye and that of the inorganic material are particles from 2 to 30 µm.

3. Process according to claim 1 or 2, characterised in that the inorganic material is a powder having particle sizes from 2 to 16 µm.

4. Process acording to any of claims 1 to 3, characterised in that the particles of the dye have sizes ranging from 5 to 10 µm.

5. Process according to any of claims 1 to 4, characterised in that the powder of the inorganic material has an apparent density not exceeding 300 $g/dm^3$ and preferably in the range from to 200 $g/dm^3$ in the non-compacted state.

6. Process according to any of the preceding claims, characterised in that the powder of the inorganic material has a specific surface below 8 $m^2/g$.

7. Process according to any of the preceding claims, characterised in that the inorganic material is an oxide or silicate of a metal, in particular zinc, titanium or silicon, aluminium, particularly a natural silicate of the kaolin, bentonite, talc type and silica.

8. Process according to any of claims 1 to 6, characterised in that the mineral compound is a microcrystalline silica which is hydrophilic or rendered hydrophobic.

9. Process according to any of the preceding claims, characterised in that the dye is an organic synthetic or minimal colouring material.

10. Process according to any of the preceding claims, characterised in that the dye is a colour-yielding plant extract.

11. Composition for carrying out the process according to any of claims 1 to 10, characterised in that it comprises an intimate mixture of 5 to 98% by weight of dry dye-stuff and respectively 95 to 2% of the inorganic material, the two components being particles from 1 to 5 µm.

12. Composition according to claim 11, characterised in that it comprises solely 10 to 80% of the dry pulverulent dye-stuff having particles from 2 to 30 µm and respectively 90 to 20% of the inorganic material having particles from 2 to 30 µm.